# EUROPEAN PATENT SPECIFICATION

(11) **EP 2 747 883 B1**
(45) Date of publication and mention of the grant of the patent: **15.02.2017**
(21) Application number: 12751504.7
(22) Date of filing: 27.08.2012
(51) Int. Cl.: B01J 14/00, C07C 51/09, C07C 53/02, B01J 10/00, B01D 3/10, C07C 51/44

(54) **PROCESS OF FORMIC ACID PRODUCTION BY HYDROLYSIS OF METHYL FORMATE**
VERFAHREN ZUR HERSTELLUNG VON AMEISENSÄURE DURCH HYDROLYSE VON METHYLFORMIAT
PROCÉDÉ DE PRODUCTION D'ACIDE FORMIQUE PAR HYDROLYSE DE FORMIATE DE MÉTHYLE

(30) Priority: 27.08.2011 US 201161528204 P
(43) Date of publication of application: 02.07.2014
(73) Proprietor: Taminco, 9000 Gent (BE)
(72) Inventor: PASEK, Josef, 160 00 Praha 6 (CZ); TREJBAL, Jiri, 278 01 Kralupy nad Vltavou (CZ); ROOSE, Peter, 9831 Sint-Martens-Latem (BE)
(74) Representative: Gevers Patents
(86) International application number: PCT/EP2012/066622
(87) International publication number: WO 2013/030162

(56) References cited:
- WO-A1-00/39067
- US-A- 4 262 140
- US-B1- 6 713 649
- US-B2- 6 696 603

## Description

### FIELD OF THE INVENTION

The present invention relates to methods and apparatus for the production of formic acid by hydrolysis of methyl formate.

The present invention further relates to methods for the removal of methanol which is formed as a side product during this production.

### BACKGROUND OF THE INVENTION

Hydrolysis of methyl formate is a dominant process of formic acid production. Formic acid of a concentration of 85 % wt. is sold predominantly on the market, only a minor part requires a concentration of 90 % and higher.

Methyl formate usually containing 3 to 8 % of methanol is typically hydrolyzed at 100 to 140 °C using water and methyl formate as starting components at a molar ratio of water/ester of between 0,8 and 7. Hydrolysis of methyl formate has an unfavorable chemical equilibrium [equilibrium constant K = 0,18 to 0,24]. In the present industrial processes, non-reacted ester and formed methanol are separated after the completion of the reaction from the reaction mixture by distillation. Formic acid of a concentration of between 20 to 50 % is obtained depending on the starting molar ratio of H₂O/ester. During rectification of the reaction mixture to dilute formic acid, very volatile methyl formate gets separated rapidly in the column, and this enables reverse esterification of formic acid by the methanol which still remains after removal of the methyl formate.
This rectification is performed at as low temperature as possible, and thus optionally at a pressure lower than atmospheric pressure (US 4299981). However, the normal b.p. of methylformate is 32 °C, and it is not possible to use common cooling water in the condenser at a reduced pressure. The extent of the reverse esterification in the rectification column can eliminate up to 20 % of formic acid that has been originally formed in the reactor.
Methyl formate that formed in the rectification column by this reverse reaction can be returned to the hydrolysis, though water formed by the esterification dilutes obtained formic acid, and it increases heat consumption.

Separation of 85 % of formic acid from diluted acid is complicated by nearly the same b.p. of water and formic acid (the normal b.p. of the acid is 101 °C) and further by formation of an azeotropic mixture with maximum of b.p. To obtain formic acid with a concentration of 85 % in the bottom of a dewatering column, it would be necessary to rectify the diluted acid at a pressure around 3,5 bar. Water with a low content of formic acid up to 1 % distillates via the top of the column, it is sent back to the hydrolysis. This procedure of isolation of formic acid of 85 % is complicated by several circumstances:
- The temperature in the bottom of the column is 150 °C at a pressure of 3,5 bar, and the extent of decomposition of formic acid to CO and water is very remarkable at this temperature. Depending on the magnitude of the liquid hold-up in the bottom of the column, 4 to 10 % of formic acid decompose. The formed water then further dilutes formic acid.
- The specific heat consumption is considerable, and the heat duty of the reboiler is around 2400 kWh/t of formic acid 85 %. This value is valid for inlet concentration of formic acid to the rectification column of 45 %.
- For the conditions of the rectification, the column, packing, distributors, reboiler and even the condenser have to be manufactured from non-metallic materials. Formic acid corrodes even the most high-quality stainless steels under the conditions of the rectification.

Formic acid production is described e. g. by patents Leonard Jackson Day US 4299981, Bethlehem Steel Corp. US 3907884. However, it is assumed by mistake in the second of the mentioned patents that formic acid forms with water an azeotrope with minimum of b.p., but it forms an azeotrope with maximum of b.p. in a matter of fact.

It has been found in the context of the present invention that the extent of formic acid decomposition can be limited by decreasing the temperature in the rectification column at the expense of decreasing the concentration of formic acid in the bottom stream of the column. Decomposition of formic acid has high activation energy, and it is strongly inhibited by the presence of water. Decreasing temperature by 10 °C and increasing water concentration from 15 % to 20 up to 25 % decreases the rate of the decomposition of the acid to one third. The acid which obtained by this method and has a concentration of 75 to 80 % is then rectified to the acid of 85 % and higher at a reduced pressure.

Formic acid can be extracted from diluted solutions to various solvents, such that water does not have to be separated from formic acid by distillation. Extraction and extraction agents based on amides of acids are described in e.g. patent publications DE2914671, US6867329, US6696603 and DE4444979. More in particular, US6696603 discloses a process for obtaining anhydrous or substantially anhydrous formic acid, in which the aqueous formic acid is supplied to a separate extraction column in which it is partly dehydrated by means of carboxylic acid amide.

Extractive rectification of a mixture of water and formic acid is also patented, which uses similar solvents as used in the above mentioned extraction methods. This process described in e.g. DE2545730, US4935100, US5006205, US5173156 and CN101481304.

There is a need to obtain improved methods for formic acid production wherein a greater conversion of methyl formate into formic acid is obtained such that heat for both distillation of non-reacted ester and for separation of water from formic acid can be saved. Unfortunately, methyl formate has the lowest b.p. (32 °C) among the all concerned components, and the higher equilibrium conversion of hydrolysis can not be achieved by separation of the product from reactor by distillation. Furthermore, formic acid can not be extracted selectively, because all solvents extract methyl formate preferably.

Prior art methods for formic acid production exist wherein weak bases are added to the reaction mixture, which bond formic acid and thus increase the conversion of methylformate. Methyl formate and methanol are then distilled from the obtained salt of formic acid with the base, and then the base is separated from formic acid at a higher temperature by distillation. Various bases are described in DE2744313. Also dialkylamides can be used to shift the equilibrium of hydrolysis DE3411384.
US 6713649 discloses a process for the preparation of formic acid, comprising extraction of aqueous formic acid with at least one formic acid ester to give a mixture of at least one formic acid ester and formic acid, and separation of at least one formic acid ester and formic acid by distillation.
WO 00/39067 discloses a method for preparing formic acid, in which methyl formate is hydrolyzed into formic acid and methanol, wherein methyl formate is fed through an ion exchange bed, in which hydrolysis to formic acid and methanol and separation of formic acid from methanol take place simultaneously.
US 4262140 discloses a process for preparing anhydrous or substantially anhydrous formic acid by hydrolysis of methyl formate in the middle section of a column, with water and methyl formate in countercurrent. The resulting formic acid is extracted, in the lower section of the column, by means of a carboxylic acid amide. The extract phase, consisting mainly of formic acid and the carboxylic acid amide, is distillatively dehydrated, or substantially dehydrated, in the lower section of the column.

### SUMMARY OF THE INVENTION

One aspect of the invention relates to methods of producing formic acid by hydrolysis of methyl formate in a reactor comprising reacting methyl formate and water in a liquid phase at a temperature of between 90-140 °C, thereby producing formic acid and methanol, and introducing a vapor stream of methyl formate during the reaction, thereby removing methanol from the reaction mixture .

The methods of the present invention rely on the principle of influencing the equilibrium of methyl formate hydrolysis via removal of methanol produced in the reaction mixture by passing (stripping) methyl formate vapours through the reaction mixture. Accordingly, methyl formate is both reactant and stripping agent.

In embodiments of these methods, the reaction is performed at a pressure of between 2 and 7 bar.
In embodiments of these methods, 4 to 8 kg of methyl formate vapor is introduced per kg of formic acid formed.
With an increasing volume of methyl formate vapours concentration of formic acid in the reaction mixture leaving the last unit of the cascade increases as well.

In embodiments of these methods, the passing of the methyl formate vapor stream is performed in a countercurrent flow.

In embodiments of these methods, the hydrolysis of methyl formate to formic acid is performed continually in a reactor-mixer cascade and wherein methyl formate vapor is fed to the last unit of the reactor-mixer cascade and wherein water is fed to the first (through the middle) reactor-mixer.
Embodiments of methods of the present invention envisage a batch design of methyl formate hydrolysis wherein methyl formate vapours are passed through the reaction mixtures at appropriate reaction temperatures and pressure until the ratio of formic acid : water of at least 2,5:1 is achieved.

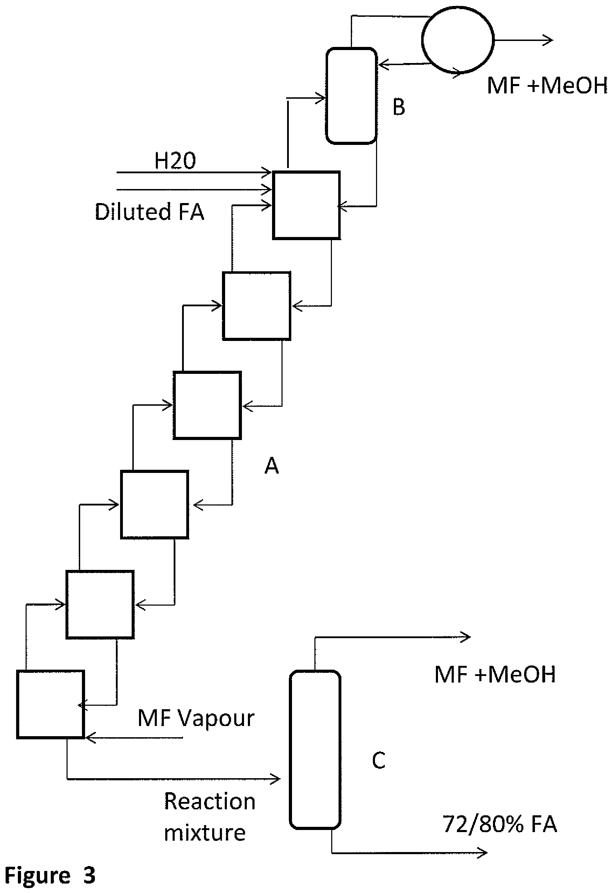

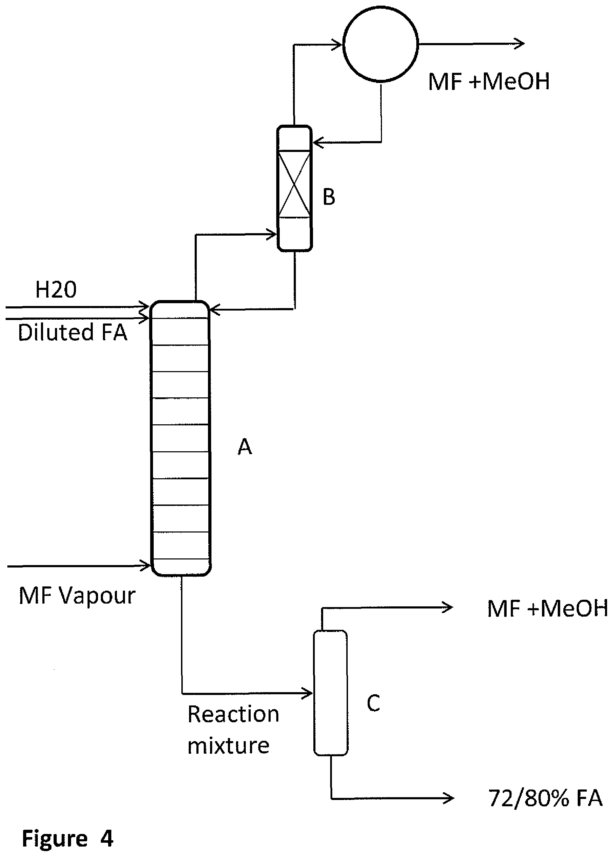

Other embodiments of methods of the present invention envisage a continuous cascade of reactors - mixers. The number of reactors - mixers may range from 4 to about 15.
A reactor-mixer cascade with a counter current flow setting can be realized in several ways.
a) a real cascade of vessels of appropriate volumes while methyl formate vapours are fed gradually from the last reactor to the first one, and vapours are fed to the bottom of each vessel, i.e. under the liquid surface by an appropriate distributor (Fig 3).
b) a reactive rectification column with cap trays constructed for a large hold up of liquid. One real tray does not correspond to one mixer in which the liquid-vapour equilibrium sets up (Fig 4).
c) a column filled with a liquid bubbled through with methyl formate vapours. In order to reduce axial mixing of the reaction mixture the bubble column is equipped with either a large number of perforated buffless or a packing.
Water is fed to the reactor-mixer cascade so that it is divided into the first through the middle unit of the cascade. All volume of water can be fed either to the first unit only or to several other units as well. If water is fed in a liquid form it is practical to feed it to the first unit. It is also practical to feed some heat to the reactor-mixer cascade as methyl formate hydrolysis is a slightly endothermic reaction. This can be realized either via heat-exchanging through the wall or feeding of water steam to the reaction mixture. In such a case water steam forms a part of water needed for methyl formate hydrolysis. However, it is not advisable to introduce water steam to the first unit, but preferably, to introduce it to other units of the cascade. For example, if the cascade has 10 units, it is advantageous to introduce steam to units 3 to 5.

Optionally,apart from water also diluted formic acid is also fed to the first reactor-mixer.
In addition to water introduced to the first and/or other units of the reactor-mixer cascade some formic acid can be fed. More particularly, an azeotropic mixture of formic acid - water from the vacuum rectification of the reaction mixture is used for this purpose.
Formic acid is a catalyst of methyl formate hydrolysis, and without presence of the acid the hydrolysis runs very slowly even at a temperature above 100 °C. There is a certain concentration of formic acid in the first unit of the reactor-mixer cascade even though the acid is not added to the reaction mixture. It is a property of a continuous flow reactor-mixer. However, an addition of formic acid to the first unit increases the reaction rate. Upon use of the acid coming off vacuum rectification with a concentration of 63 to 66 % of formic acid, the contained acid is a catalyst and the contained water is a reactant. Thus the relatively diluted acid in the reactor is thickened to a concentration of 72 to 80 %.

In embodiments of these methods, methylformate vapor stream is introduced in the reactor at a pressure of between 2 and 7 bar.

In further embodiments these methods, comprise the step of c) separating methanol and methyl formate.
Typically, small quantity of formic acid is separated from methanol and methyl formate vapours via partial condensation, wherein methanol is separated from the remaining gaseous mixture of methyl formate and methanol, and wherein methyl formate is recycled to the last unit of the reactor-mixer cascade.
Generally, vapours leaving the reactor can be directly relieved of methanol without separation of a small quantity of formic acid, which is present in the vapours. However, if the formic acid is not separated from the vapours it complicates separation of methanol from methyl formate as acid catalyses both hydrolysis of methyl formate and esterification of methanol with formic acid. For separation of methanol from methyl formate vapours via extractive rectification water is an optimal solvent. However, in the presence of formic acid water could not be used because methyl formate in the extractive rectification column would hydrolyze. The presence of acid also leads to considerable corrosion.
Formic acid is eliminated from the mixture of vapours using partial condensation associated with certain, relatively small rectification efficiency. This task is preferably resolved by a small rectification column with an efficiency of 8 to 10 theoretical plates refluxed with a partial condenser. Liquid flows out of the column bottom back to the reactor, i.e. the first unit of the cascade. A similar purpose is also achieved using three back-flow condensers in series.
Methanol is to be removed from the mixture of methyl formate and methanol vapours. Both substances form an azeotropic mixture with the minimum boiling point, and even an efficient rectification can produce methyl formate containing 3 % of methanol. It is also possible to separate methyl formate and methanol using a membrane as well.

In these embodiments methanol and methyl formate are typically separated by extractive distillation with a polar solvent, such as water,
a glycol, a glycol ether, and an amide of a short-chain fatty acid.

The methods of the present invention may comprise a step in which methanol is removed from the flow of methyl formate vapours, concurrently both from the vapours exiting the first unit of the reactor-mixer cascade and the flow of vapours of fresh (stoichiometric) methyl formate and the flow of vapours of methyl formate separated from the reaction mixture.
In the methods of the invention a large volume of methyl formate vapours circulates between the reactor and the extractive rectification column. Methyl formate distilled off the reaction mixture is also returned to the process; it contains up to 1 -2% of methanol. In addition, fresh methyl formate (stoichiometric) is introduced to the process, the quantity of which corresponds to the formic acid produced. This fresh methyl formate is produced via carbonylation of methanol and it contains 4 to 6 % of methanol.
Methanol is to be eliminated from the two flows of methyl formate as well and it can be advantageously performed simultaneously with the main flow of methyl formate using extractive rectification.

Further embodiments comprise the step of adding the separated methylformate to the reactor.
Methyl formate relieved of methanol through extractive rectification typically condensed and recycled to the reactor as a liquid using a pump. Before entering the reactor methyl formate is evaporated. From an energetic point of view, it is practical to condense reflux of the extractive rectification column only (a partial condenser) and recycle methyl formate to the reactor in as a vapour using a compression device. The consumed compression work has a value of about 10 % of the steam value which would be used for evaporation of condensed methyl formate.

In further embodiments of the methods of this aspect of the invention the hydrolysis of methyl formate and concurrent removal of methanol from the reaction mixture in a flow of methyl formate vapours is performed at a pressure of between 2 to 7 bar.
Operating pressure in the reactor depends from the temperature. For example, at the maximum temperature 140 °C methyl formate vapour tension is 17 bar. Even at a pressure slightly lower than the dew point of methyl formate the content of methyl formate in a liquid reaction mixture would be too high and at pressure of e.g. 10 bar the process according to the invention cannot be realized. The optimum pressure in the entry of methyl formate to the reactor-mixer cascade is generally 4 to 5 bar which applies to the temperature in the last unit of the cascade (having the highest temperature) of 125 to 130 °C.
At reaction temperature higher than 140 °C higher pressures could be achieved. From 140 °C onwards formic acid degrades into CO + H₂O. Generally, temperatures above 130 °C are not used.

Typically, the reaction is performed until a reaction product is obtained with a mass ratio of water/formic acid between to 1 / 2.5 and 1/ 4 and whereby the reaction product is withdrawn from the reactor.
For example, methylformate and methanol are removed by rectification from the reaction product and remaining water-formic acid mixture has a mass ratio of water/formic acid between to 1 / 2.5 and 1/4.

In further embodiments of this aspect of the invention the methods further comprising the step of concentrating formic acid.

Typically formic acid is separated from methyl formate by distillation until formic acid is obtained at a concentration of 85 to 90 %.

Such a distillation can be performed at a pressure of between 8 and 20 kPa, particularly at a pressure between 10 and 13 kPa.

According to certain embodiments of the invention a side product of 63-66 % formic acid from the distillation is delivered to the reactor of claim 1.

In these methods, methyl formate and methanol are removed by rectification from the reaction mixture containing formic acid, water and small amounts of methanol which are returned to the process. The remaining quantity of water and formic acid containing minimum 70 % of formic acid is processed to acid of concentration 85 to 99 wt% by vacuum rectification.
The reaction mixture coming off the last unit of the cascade (from the reactor in general) contains formic acid, water, methyl formate and a small volume of methanol. With the method as described herein, an acid of a concentration of 75 to 80 % is obtained, from which formic acid of any concentration is prepared via vacuum rectification. Commercial formic acid is mainly 85% formic acid and to a smaller extent 99% formic acid.
The final vacuum rectification at a temperature of max. 60 °C is provides an inventive aspect. In the prior art, formic acid is rectified at a relatively high temperature, whereby a certain amount of formic acid always degrades. Moreover, formic acid has strong corrosive effects and corrosion rate increases rapidly with increasing temperature. In vacuum low-temperature rectification of formic acid common types of stainless steel can be used; rectification at a relatively high temperature requires expensive special materials.

A further aspect of the invention relates to the use of gaseous methylformate for removing methanol during a reaction wherein formic acid is produced by hydrolysis of methyl formate.

A further aspect of the invention relates to apparatus for the production of formic acid comprising :
- a reactor (A) suitable for the conversion of methylformate into formic acid comprising means for the delivery of water, formic acid and methyl fomate, and further comprising means for collecting formic acid at the bottom part of the reactor and means for collecting methanol and methyl formate at the top part of the reactor,
- a rectification column (C) suitable for the separation of methanol and methyl formate by extractive rectification,
wherein the reactor A is fluidly connected with a rectification column C for receiving methanol and methyl formate collected at the top part of the reactor (A), the rectification column further comprising means for the delivery of a polar solvent, means for the collection of methanol at the bottom part of the rectification colum and means for the collection of methyl formate at the top of the rectification column.

Typically, the means for the collection of methyl formate at the top of the rectification column (C) are fluidly connected with the means for of methyl fomate into reactor (A).
In specific embodiments, the fluidic connection between the top of rectification column (C) and the bottom of reactor (A) comprises a compressor.

In specific embodiments, the fluidic connection between the top of reactor A and rectification column C comprises a rectification vessel (A') for the removing of formic acid from the vapours leaving reactor A

The method of the present invention have the advantage that the reaction mixture contains minimum 72 % of formic acid (28 % of water), i.e. the content of formic acid is higher than the composition of azeotrope with the maximum boiling point of formic acid - water at a pressure of 10 kPa, which is 63 to 64 %. The process allows us to produce formic acid in reactor, at a concentration as high as 80 %. The most of formic acid sold in the world market has the concentration of 85 % of acid and the acid is obtained from the reaction mixture easily via vacuum rectification. However, formic acid produced out of the reaction mixture via vacuum rectification may have the concentration around 99 %.

### BRIEF DESCRIPTION OF THE FIGURES

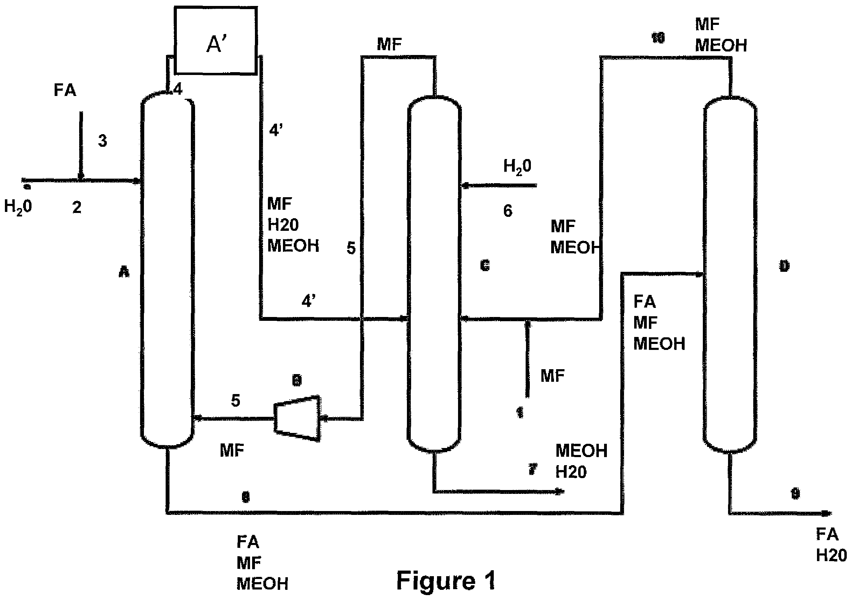

Figure 1 show a schematic representation of an embodiment of methods of present invention. The abbreviations used are FA: formic acid; MEOH: methanol; MF : methylformate ; H₂O : water.
   A: is the reaction chamber wherein methyl formate is converted into formic acid.
   A': is the partial reflux condenser wherein formic acid is separated from methyl formate/water/methanol vapours.
   B: compressor.
   C: is the rectification column wherein methanol is separated from methyl formate.
   D: is the rectification column wherein formic acid- water mixture is separated from methyl formate and methanol.

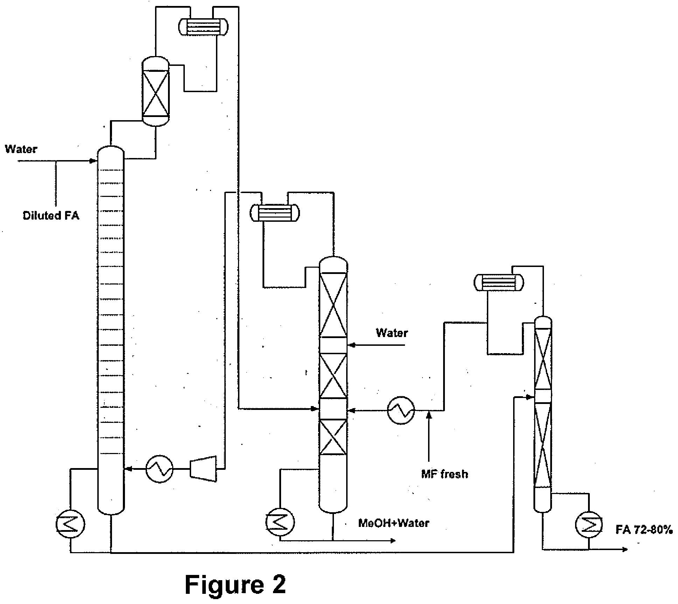

Figure 2 shows an alternative presentation of the embodiment of figure 1.
Figure 3 and 4 show exemplary arrangements of counter-current cascades of mixers-reactors.

### DETAILED DESCRIPTION OF THE INVENTION

One embodiment of the present invention relates to a method of methyl formate hydrolysis by water at a temperature of 90 to 140 °C, comprising separation of formed methanol during the reaction by stripping the reaction mixture with methyl formate gas in a counter current reactor at a pressure of 3 to 6 bar, such that methanol is removed from the reaction mixture. The stripping process thus results in the release of a gas which contains methyl formate, methanol, water and formic acid.

Water and formic acid are separated from this gas. The remaining methanol and methyl formate are separated by extractive distillation by adding a polar solvent. This extractive method can also be used to remove methanol from fresh methyl formate which is typically contaminated with about 3 to 8 of methanol.

The column for extractive distillation can be fed by introducing at the same moment the recycled methanol/methyl formate gas from the stripping process as well as the vaporized fresh methyl formate and non reacted recycled methyl formate.

The methyl formate vapors from the column of extractive rectification are introduced by a compressor to the bottom of the counter current reactor.

The reaction of producing formic acid is performed until a reaction mixture can be withdrawn from the reactor bottom with a mass ratio of water/formic acid = 1/2,5 to 1/4 . Methyl formate and a small amount of methanol which are present in the withdrawn reaction mixture are separated by rectification.. A formic acid concentration of 85 % or higher is obtained by a well-known rectification procedure of choice.

The counter-current reactor (A) can be a counter-current cascade of reactors mixers (CSTR). The inner wall of this reactor is typically made of enameled steel. Water (2) enters the first mixer of the reactor, and vapors (4) of methanol, methyl formate, water and small amount formic acid exit it. Water and formic acid can be best separated from these vapors in a rectification column with a partial reflux condenser (A'). Then only a mixture of vapors of methyl formate with methanol (4') exits the partial condenser. These substances form an azeotrope containing 7% methanol at a pressure of 4 bar, which is beneficial for stripping of methanol from the reaction mixture. Superheated vapors of methyl formate are routed to the last mixer, they proceed from the last reactor to the first one. The number of the mixers in the cascade shall not be too little, a suitable number is from 4 to 15.

A suitable arrangement of the counter-current reactor (A) is a reactive column, whose bottom superheated vapors of methyl formate (5) enter, and a mixture of vapors (4) of methyl formate, methanol, water and small amount of formic acid are withdrawn from the top of the reactive column. Formic acid and water are separated from this mixture in a rectification column refluxed by a partial condenser (A'). Only methyl formate and methanol vapors containing small amount water (4') exit the condenser.

Methanol has to be separated from the gas mixture of methylformate and methanol. Partial condensation is not suitable with respect to the concentration close to the azeotropic mixture. According the invention extractive distillation is thus beneficial, when methanol is extracted by a polar solvent. Namely water has suitable properties for this purpose, then dimethylformamide, ethylene glycol and others can be used too. Methanol is washed out in the middle part of the column (C) by the solvent (6), the solvent is separated from methyl formate in the upper part of the column, and methyl formate is separated from the mixture of methanol with the solvent in the bottom part (7) of the column. Methyl formate vapors (5) exit the partial condenser of the extractive rectification column, and they are routed back to the counter-current reactor (A).

Methyl formate vapors (5) are routed back to the counter-current reactor by a compressor (B). The compressor operates at a temperature around 60-80 °C. With respect to a low compression ratio, the consumption of the work of compression is low.

The reaction mixture (9) is withdrawn from the bottom of the counter-current reactor (A), the reaction mixture has a mass ratio of water / formic acid = 1 / 2,5 to 1 / 4. Methyl formate and a small amount of methanol can be easily separated from this mixture in rectification column (D). For this rectification, the risk of the reverse esterification is very low, because the mixture contains only very little methanol (1,0 to 3 %). Formic acid (9) at a concentration of 72 to 80 %, with advantage at a concentration of 75 to 77 %, is obtained from the bottom of the column. Formic acid of 85 % and optionally even of a higher concentration is obtained by vacuum rectification using methods known to the skilled person. A suitable pressure of such prior art rectification is 8 to 20 kPa, preferably 10 to 12 kPa.

Formic acid with a concentration of 63 to 66 % (9) is withdrawn from the bottom of the vacuum column (D). The acid can be routed back (3) to the counter-current reactor for concentrating up advantageously. Returned formic acid has a catalytic effect on hydrolysis of methyl formate. It is possible to remove water from this acid prior to the introduction in the reaction column A, by rectification at a pressure of 2 to 2,5 bar and to add the obtained acid with a concentration of 75 to 80 % to the vacuum rectification. If the procedure according to this invention is followed, then only a small amount of water is evaporated.

The process of formic acid production according to the invention produces formic acid with a beneficial ratio of water - acid in the reactor, 72-80% formic acid. Furthermore, the pressure in the reactor (A) of methyl formate hydrolysis is max. 7 bar which is lower than in the prior art processes where it is from 5 up to 18 bar. The procedure according to the invention does not include addition of foreign substances to the reactor such as bases, which can react there. When dimethylformamide (DMF) is used instead of water as extractive agents (6) in column, it does not come into contact with the acid, and thus it can not hydrolyze. The DMF is removed with the MeOH and does not enter the reaction process.

A big problem of the classical rectification of a mixture of water with formic acid at a pressure above 3 bar is severe corrosion. A quite large column, its packing, even its reboiler has to be manufactured from special expensive materials. In the process according to the invention, the use of such materials is limited only to the reactor (A) and two relatively small columns.

In the process according to the invention, there are no conditions for a greater extent of the reverse esterification. The temperature in the rectification column (D) and further columns, where formic acid is present, is 115 °C. Formic acid does not decompose to CO and H₂O since this decomposition of formic acid takes place at temperatures above 140 °C.

The total consumption of heat for production of formic acid of 85% is 2,5 t of steam / 1 t of formic acid 85 %, and thus it is lower than for the conventional process of hydrolysis and isolation of formic acid of 85 % by rectification, and it is a little lower to the extraction process of methylformate hydrolysis, but the process according to the invention is not complicated by side reactions, like reverse esterification, decomposition of formic acid at a high temperature and chemical changes of the solvent in the reactor and other parts of the process.

The methods and apparatus of the present invention allow to perform a continuous process, wherein a reduction in pressure is compensated by the compressor.

The principle of the stripping process for the production of formic acid by the hydrolysis of methyl formate according to the invention will be explained in a simplified process flow diagram.
The reactive column A is equipped with rectifying trays, on which the methyl formate hydrolysis takes place and the methanol formed in the reaction mixture is stripped off by methyl formate vapours at the same time. Water (Stream 2) and diluted formic acid from the reaction product rectification (Stream 3) are brought into the upper section of the reactive column A . Formic acid at a concentration of about 65% catalyses the hydrolysis of methyl formate and, at the same time in this process, it concentrates itself in the column reactor up to 72 to 80%.
From compressor B vapours of methyl formate are fed to the bottom of the reactive column A . The stream of the methyl formate vapours consists of gaseous methyl formate leaving the column C head (Stream 5).
A mixture of methyl formate, methanol, water and small amount of formic acid leaves the top section of the reactive column A. In the column A', which is refluxed with the liquid formed in the partial condenser, formic acid is removed from these vapours.The uncondensed mixture of methyl formate, methanol and a small portion of water is brought into the extractive rectification column C (Stream 4'). In the column C, polar methanol is extracted by water from the mixture (Stream 6). The upper section of the column C is refluxed by the partial condenser and methyl formate vapours purified from methanol (down to 0,1%) are recycled by the compressor B back to the bottom of the reactive column A (stream 5). The mixture of vaporised fresh methyl formate (stream 1) and methyl formate regenerated in the column D (Stream 10) is added to the stream of methyl formate recycled vapours (Stream 4) to be purified from methanol too.

The hydrolysis of methyl formate to formic acid takes place in the reactive column A at 90 to 1400C, which - with regards to the vapour tension of substances, namely that of methyl formate - requires the pressure level of 2 to 7 bar. The trays of the reactive column A show a significant pressure loss and certain pressure losses occur also in the column C, heat exchangers and the piping system. The compressor B for recycling of methanol vapours overcomes the total pressure drop of approx. 1 to 1.5 bar. The vapours enter the compressor B at 60 to 800C, depending on the working pressure of the system.

In the bottom section of the extractive rectification colum C, the water-methanol mixture is freed from methyl formate and liquid methanol and water is withdrawn from the column bottom (Stream 7). for further processing by rectification methods known in the art.

From the bottom of the reactive column A, a mixture of formic acid, water, methyl formate and a small portion of methanol is withdrawn (Stream 8). In the rectification column D, this mixture is freed from methyl formate and methanol (Stream 10) and then it is returned, along with the fresh methyl formate vapours, to the column C. From the column D bottom, the mixture - containing 72 to 80% of formic acid (the rest is water) - is drawn off (Stream 9). Subsequently, this mixture is processed via conventional vacuum rectification to formic acid at a concentration of at least 85% and diluted formic acid (about 63-66%), where the latter is returned to the reactive column A (Stream 3).

### EXAMPLES

### Example 1. Methyl formate hydrolysis with stripping of methanol.

Symbols as used in Figure 1 are used to indicate the different elements and flows.

Hydrolysis of methyl formate is carried out in an enameled tube (A) with a diameter of 5 cm and a length of 3 m equipped with 10 perforated baffles from fluoropolymer. This tube reactor is tempered by steam with condensation temperature of 130 °C. A mixture of water (2) and 60-65 % formic acid (3) preheated to 110 °C is fed to the top of the reactor in an amount of 1400 g/h of water and 820 g/h of formic acid.

Methyl formate is fed (5) to the bottom of the tube reactor in the form of vapors at a temperature of 140 °C in an amount of 8500 g/h. Reactor (A) operates at a pressure of 4 bar, the bottom temperature is 127°C, the top temperature is 118 oC

An enameled rectification vessel (A') partially packed with ceramic saddles of 4 mm and a bed height of 1 m is placed above the reactor (A), and receives the methyl formate, methanol, water and formic acid mixture (4). The upper part of the enameled tube above the packing is cooled down in the shell by water, whereby the amount of cooling is adjusted such that the amount of formic acid in the vapour stream leaving the rectification vessel A' is not more than about 10 ppm .

Vapors (4') are routed to the column (C) of extractive rectification, which has a diameter of 40 mm, and it has 3 beds of Sulzer DX gauze packing, the bed height (from the bottom to the top) is 60, 60 and 40 cm. The gas mixture (4') of methyl formate, methanol and water from the partial condenser (A') where it has been freed from formic acid and a part of the water is fed above the 1 st packed bed, distilled water (6) at a temperature of 50 °C is fed above the second bed.

Column C operates at the same pressure as reactor A (4 bar), and condensed methyl formate is partly refluxed and partly fed (5) to the bottom of the tube reactor. In this way, methyl formate which left the reaction column via the extractive rectification has been recycled and can be reintroduced into the reaction column.

Column C is also used to remove methanol from fresh methyl formate. Fresh liquid methyl formate (1) is evaporated and added in an amount of 4000 g/h into the extractive rectification column.

The present experimental setting makes no use of a compressor as depicted in the drawing and foreseen in a large scale process, whereby both recycled methylformate from the stripping and fresh methylformate, freed from methanol, are introduced under pressure into the reaction column via feed 5. The absence of the compressor in this experimental setting has no effect on the concept of the present invention.

A liquid reaction mixture is withdrawn (8) from the bottom of the enameled reactor (A), which keeps the liquid level in the upper part of the reactor stable. The withdrawn liquid is fed to a glass rectification column (D) packed with two beds of ceramic packing with a height of 60 and 60 cm. Formic acid with a concentration of 75 % (9), the rest is water, is withdrawn from the reboiler. Methyl formate with a small amount of methanole is going from the top of the column D.

Formic acid of 85 to 90 % is produced from this acid (75%) by vacuum rectification at a pressure of 10 kPa in the amount 2000 g/h. The bottom residue of this rectification containing around 65 % FA can be sent back with water that is fed to the top of the counter-current reactor.

## Claims

1. A method of producing formic acid by hydrolysis of methyl formate in a reactor comprising
reacting methyl formate and water in a liquid phase at a temperature of between 90-140 °C, thereby producing formic acid and methanol, and
introducing a vapor stream of methyl formate during the reaction thereby removing methanol from the reaction mixture.

2. The method according to claim 1, wherein the reaction is performed at a pressure of between 2 and 7 bar.

3. The method according to claim 1 or 2, wherein 4 to 8 kg of methyl formate vapor is introduced per kg of formic acid formed.

4. The method according to claim 1, wherein the passing of the methyl formate vapor stream is performed in a countercurrent flow.

5. The method according to claim 1, wherein the hydrolysis of methyl formate to formic acid is performed continually in a reactor-mixer cascade and wherein methyl formate vapor is fed to the last unit of the reactor-mixer cascade and wherein water is fed to the first through the middle reactor-mixer and wherein apart from water diluted formic acid is also fed to the first reactor-mixer.

6. The method according to any one of claims 1 to 5, further comprising the step of separating methanol and methyl formate.

7. The method according to any one of claim 1 to 6, wherein formic acid is separated from methanol and methyl formate via partial condensation, wherein methanol is separated from the gaseous mixture of methyl formate and methanol, and wherein methyl formate is recycled to the last unit of the reactor-mixer cascade.

8. The method according to claim 6 or 7, wherein methanol and methyl formate are separated by extractive distillation with a polar solvent, wherein the polar solvent is water, or wherein the polar solvent is selected from the group consisting of a glycol ether, and an amide of a short chain fatty acid, and wherein a side product of 60- 65 % formic acid from the distillation is delivered to the reactor of claim 1.

9. The method according to claim 7 or 8, further comprising the step of adding the separated methyl formate to the reactor of claim 1.

10. The method according to any one of claims 1 to 9, wherein the hydrolysis of methyl formate and concurrent removal of methanol from the reaction mixture in a flow of methyl formate vapours is performed at a pressure of between 2 to 7 bar.

11. The method according to claim 1, which is performed until a reaction product is obtained with a mass ratio of water/formic acid between to 1 / 2.5 and 1/ 4 and whereby said reaction product is withdrawn from the reactor.

12. The method according to claim 11 whereby, from said reaction product with a mass ratio of water/formic acid between to 1 / 2.5 and 1/ 4, methyl formate and methanol are removed by rectification.

13. The method according to claim 12, further comprising the step of concentrating formic acid.

14. Use of gaseous methyl formate for removing methanol during a reaction wherein formic acid is produced by hydrolysis of methyl formate.

15. An apparatus for the production of formic acid comprising :
- a reactor (A) suitable for the conversion of methyl formate into formic acid comprising means for the delivery of water, formic acid and methyl formate, and further comprising means for collecting formic acid at the bottom part of the reactor and means for collecting methanol and methyl formate at the top part of the reactor,
a rectification column (C) suitable for the separation of methanol and methyl formate,
wherein the reactor A is fluidly connected with a rectification column C for receiving methanol and methyl formate collected at the top part of the reactor (A), the rectification column further comprising means for the delivery of a polar solvent, means for the collection of methanol at the bottom part of the rectification column and means for the collection of methyl formate at the top of the rectification column,
and wherein a fluidic connection between the top of rectification column (C) and the bottom of reactor (A) comprises a compressor.

## Patentansprüche

1. Ein Verfahren zur Herstellung von Ameisensäure durch Hydrolyse von Methylformiat in einem Reaktor, welches Folgendes umfasst:
Reagieren von Methylformiat und Wasser in einer flüssigen Phase bei einer Temperatur von zwischen 90-140 °C, wodurch Ameisensäure und Methanol hergestellt werden, und
Einleiten eines Dampfstromes von Methylformiat während der Reaktion, wodurch Methanol aus der Reaktionsmischung entfernt wird.

2. Das Verfahren nach Anspruch 1, wobei die Reaktion bei einem Druck von zwischen 2 und 7 bar ausgeführt wird.

3. Das Verfahren nach Anspruch 1 oder 2, wobei 4 bis 8 kg Methylformiatdampf pro kg gebildeter Ameisensäure eingeleitet werden.

4. Das Verfahren nach Anspruch 1, wobei das Durchführen des Methylformiatdampfstromes in einem Gegenstrom ausgeführt wird.

5. Das Verfahren nach Anspruch 1, wobei die Hydrolyse von Methylformiat in Ameisensäure kontinuierlich in einer Reaktor-Mischer-Kaskade ausgeführt wird und wobei Methylformiatdampf der letzten Einheit der Reaktor-Mischer-Kaskade zugeführt wird und wobei Wasser durch den mittleren Reaktor-Mischer dem Ersten zugeführt wird und wobei dem ersten Reaktor-Mischer abgesehen von Wasser auch verdünnte Ameisensäure zugeführt wird.

6. Das Verfahren nach irgendeinem der Ansprüche 1 bis 5, welches ferner den Schritt des Trennens von Methanol und Methylformiat umfasst.

7. Das Verfahren nach irgendeinem der Ansprüche 1 bis 6, wobei Ameisensäure von Methanol und Methylformiat über Teilkondensation getrennt wird, wobei Methanol von der gasförmigen Mischung von Methylformiat und Methanol getrennt wird, und wobei Methylformiat zur letzten Einheit der Reaktor-Mischer-Kaskade zurückgeführt wird.

8. Das Verfahren nach Anspruch 6 oder 7, wobei Methanol und Methylformiat durch extraktive Destillation mit einem polaren Lösungsmittel getrennt werden, wobei das polare Lösungsmittel Wasser ist, oder wobei das polare Lösungsmittel aus der Gruppe bestehend aus einem Glycolether, und einem Amid einer kurzkettigen Fettsäure ausgewählt wird, und wobei ein Nebenprodukt von 60- 65 % Ameisensäure aus der Destillation zum Reaktor aus Anspruch 1 geführt wird.

9. Das Verfahren nach Anspruch 7 oder 8, welches ferner den Schritt des Zusetzens des getrennten Methylformiats zum Reaktor aus Anspruch 1 umfasst.

10. Das Verfahren nach irgendeinem der Ansprüche 1 bis 9, wobei die Hydrolyse von Methylformiat und die gleichzeitige Entfernung von Methanol aus der Reaktionsmischung in einem Strom von Methylformiatdämpfen bei einem Druck von zwischen 2 und 7 bar ausgeführt werden.

11. Das Verfahren nach Anspruch 1, das ausgeführt wird, bis ein Reaktionsprodukt mit einem Massenverhältnis von Wasser/Ameisensäure zwischen 1 / 2,5 und 1/ 4 erhalten wird und wobei das erwähnte Reaktionsprodukt aus dem Reaktor entnommen wird.

12. Das Verfahren nach Anspruch 11, wobei, aus dem erwähnten Reaktionsprodukt mit einem Massenverhältnis von Wasser/Ameisensäure zwischen 1 / 2,5 und 1/ 4, Methylformiat und Methanol durch Rektifikation entfernt werden.

13. Das Verfahren nach Anspruch 12, welches ferner den Schritt des Konzentrierens der Ameisensäure umfasst.

14. Verwendung von gasförmigem Methylformiat zum Entfernen von Methanol während einer Reaktion, wobei Ameisensäure durch Hydrolyse von Methylformiat hergestellt wird.

15. Ein Apparat zur Herstellung von Ameisensäure, welcher Folgendes umfasst:
- einen Reaktor (A) geeignet für die Umwandlung von Methylformiat in Ameisensäure, der Mittel für die Lieferung von Wasser, Ameisensäure und Methylformiat umfasst, und der ferner Mittel zum Entnehmen von Ameisensäure am unteren Teil des Reaktors und Mittel zum Entnehmen von Methanol und Methylformiat am oberen Teil des Reaktors umfasst,
eine Rektifikationskolonne (C) geeignet für die Trennung von Methanol und Methylformiat,
wobei Reaktor A fließend mit einer Rektifikationskolonne C verbunden ist, um Methanol und Methylformiat zu empfangen, die am oberen Teil von Reaktor (A) entnommen werden, wobei die Rektifikationskolonne ferner Mittel für die Lieferung eines polaren Lösungsmittels, Mittel für die Entnahme von Methanol am unteren Teil der Rektifikationskolonne und Mittel für die Entnahme von Methylformiat oben an der Rektifikationskolonne umfasst,
und wobei eine fluidische Verbindung zwischen der Oberseite der Rektifikationskolonne (C) und der Unterseite von Reaktor (A) einen Kompressor umfasst.

## Revendications

1. Procédé de production d'acide formique par hydrolyse de formiate de méthyle dans un réacteur comprenant
la mise en réaction de formiate de méthyle et d'eau dans une phase liquide à une température entre 90 et 140 °C, produisant de ce fait de l'acide formique et du méthanol, et
l'introduction d'un courant de vapeur de formiate de méthyle pendant la réaction, enlevant de ce fait le méthanol du mélange de réaction.

2. Procédé selon la revendication 1, dans lequel la réaction est réalisée à une pression entre 2 et 7 bars.

3. Procédé selon la revendication 1 ou 2, dans lequel 4 à 8 kg de vapeur de formiate de méthyle sont introduits par kg d'acide formique formé.

4. Procédé selon la revendication 1, dans lequel le passage du courant de vapeur de formiate de méthyle est réalisé dans un écoulement à contre-courant.

5. Procédé selon la revendication 1, dans lequel l'hydrolyse de formiate de méthyle en acide formique est réalisée continuellement dans une cascade de réacteurs-mélangeurs et dans lequel de la vapeur de formiate de méthyle est amenée vers la dernière unité de la cascade de réacteurs-mélangeurs et dans lequel de l'eau est amenée vers le premier réacteur-mélangeur à travers le réacteur-mélangeur du milieu et dans lequel, mise à part l'eau, de l'acide formique dilué est également amené vers le premier réacteur-mélangeur.

6. Procédé selon l'une quelconque des revendications 1 à 5, comprenant en outre l'étape de séparation de méthanol et de formiate de méthyle.

7. Procédé selon l'une quelconque des revendications 1 à 6, dans lequel de l'acide formique est séparé du méthanol et du formiate de méthyle via une condensation partielle, dans lequel le méthanol est séparé du mélange gazeux de formiate de méthyle et de méthanol, et dans lequel du formiate de méthyle est recyclé vers la dernière unité de la cascade de réacteurs-mélangeurs.

8. Procédé selon la revendication 6 ou 7, dans lequel le méthanol et le formiate de méthyle sont séparés par distillation extractive avec un solvant polaire, dans lequel le solvant polaire est de l'eau, ou dans lequel le solvant polaire est choisi dans le groupe consistant en un glycol éther, et un amide d'un acide gras à courte chaîne, et dans lequel un produit secondaire d'acide formique à 60 à 65 % issu de la distillation est délivré au réacteur de la revendication 1.

9. Procédé selon la revendication 7 ou 8, comprenant en outre l'étape d'addition du formiate de méthyle séparé au réacteur de la revendication 1.

10. Procédé selon l'une quelconque des revendications 1 à 9, dans lequel l'hydrolyse de formiate de méthyle et l'élimination par cocourant de méthanol du mélange de réaction dans un écoulement de vapeurs de formiate de méthyle sont réalisées à une pression entre 2 et 7 bars.

11. Procédé selon la revendication 1, qui est réalisé jusqu'à ce qu'un produit de réaction soit obtenu avec un rapport massique eau/acide formique entre 1/2,5 et 1/4 et moyennant quoi ledit produit de réaction est retiré du réacteur.

12. Procédé selon la revendication 11, dans lequel, à partir dudit produit de réaction avec un rapport massique eau/acide formique entre 1/2,5 et 1/4, du formiate de méthyle et du méthanol sont éliminés par rectification.

13. Procédé selon la revendication 12, comprenant en outre l'étape de concentration d'acide formique.

14. Utilisation de formiate de méthyle gazeux pour éliminer du méthanol pendant une réaction dans laquelle l'acide formique est produit par l'hydrolyse de formiate de méthyle.

15. Appareil de production d'acide formique comprenant :
- un réacteur (A) convenant à la conversion de formiate de méthyle en acide formique comprenant un moyen de délivrance d'eau, d'acide formique et de formiate de méthyle, et comprenant en outre un moyen de collecte d'acide formique à la partie basse du réacteur et un moyen de collecte de méthanol et de formiate de méthyle à la partie haute du réacteur,
- une colonne de rectification (C) convenant à la séparation de méthanol et de formiate de méthyle,
dans lequel le réacteur A est raccordé fluidiquement avec une colonne de rectification C destinée à recevoir du méthanol et du formiate de méthyle collectés à la partie haute du réacteur (A), la colonne de rectification comprenant en outre un moyen de délivrance d'un solvant polaire, un moyen de collecte de méthanol à la partie basse de la colonne de rectification et un moyen de collecte de formiate de méthyle au haut de la colonne de rectification,
et dans lequel un raccordement fluidique entre le haut de la colonne de rectification (C) et le bas du réacteur (A) comprend un compresseur.
